# EUROPEAN PATENT SPECIFICATION

(11) **EP 1 227 843 B1**
(45) Date of publication and mention of the grant of the patent: **14.10.2009**
(21) Application number: 00992499.4
(22) Date of filing: 10.11.2000
(51) Int. Cl.: A61K 39/395, C07K 1/22, A61P 31/00, A61P 35/00, A61P 37/00, C07K 16/28, C07K 14/715

(54) **METHOD AND SYSTEM TO REMOVE CYTOKINE INHIBITOR IN PATIENTS**
VERFAHREN UND SYSTEM ZUR ENTFERNUNG VON ZYTOKININHIBITOREN IN PATIENTEN
METHODE ET SYSTEME PERMETTANT DE SUPPRIMER L'INHIBITEUR DE CYTOKINES CHEZ DES PATIENTS

(30) Priority: 10.11.1999 US 164695 P
(43) Date of publication of application: 07.08.2002
(60) Divisional application: 09005155.8
(73) Proprietor: BioPheresis Technologies, Inc., Atlanta, GA 30339 (US)
(72) Inventor: Lentz, M. Rigdon, 83209 Prien am Chiemsee (DE)
(74) Representative: Lahrtz, Fritz
(86) International application number: PCT/US2000/042090
(87) International publication number: WO 2001/037873

(56) References cited:
- WO-A-99/61085
- US-A- 4 708 713
- C. SELINSKY ET AL.: "Multifaceted inhibition of anti-tumour immune mechanisms by soluble tumour necrosis factor receptor type I." IMMUNOLOGY, vol. 94, no. 1, May 1998 (1998-05), pages 88-93, XP002172135 Oxford, GB
- M. LENTZ: "The role of therapeutic apheresis in the treatment of cancer: a review." THERAPEUTIC APHERESIS, vol. 3, no. 1, February 1999 (1999-02), pages 40-49, XP001010150 USA
- M. LENTZ: "Continuous whole blood UltraPheresis procedure in patients with metastatic cancer." JOURNAL OF BIOLOGICAL RESPONSE MODIFIERS, vol. 8, no. 5, October 1989 (1989-10), pages 511-527, XP001010099 New York, NY, USA
- T. GATANAGA ET AL.: "Purification and characterization of an inhibitor (soluble tumor necrosis factor receptor) for tumor necrosis factor and lymphotoxin obtained from the serum ultrafiltrates of human cancer patients." PROCEEDINGS OF THE NATIONAL ACADEMY OF THE U.S.A., vol. 87, no. 22, November 1990 (1990-11), pages 8781-8784, XP002172137 Washington, DC, USA
- C. TETTA ET AL.: "Continuous plasma filtration coupled with sorbents." KIDNEY INTERNATIONAL, vol. 53, no. suppl. 66, May 1998 (1998-05), pages S186-S189, XP001010123 New York, NY, USA

## Description

### Background of the Invention

The present invention is generally in the field of enhancing an immune response, and particularly relates to the removal of TNF inhibitors in a patient, such as a cancer patient, to promote inflammation and thereby induce remission of the cancer.

Conventional cancer therapy is based on the use of drugs and/or radiation which kills replicating cells, hopefully faster than the agents kill the patient's normal cells. Surgery is used to reduce tumor bulk, but has little impact once the cancer has metastasized. Radiation is effective only in a localized area.

The treatments can in themselves kill the patient, in the absence of maintenance therapy. For example, for some types of cancer, bone marrow transplants have been used to maintain the patient following treatment with otherwise fatal amounts of chemotherapy. Efficacy has not been proven for treatment of solid tumors, however. "Cocktails" of different chemotherapeutic agents and combinations of very high doses of chemotherapy with restorative agents, for example, granulocyte macrophage colony stimulating factor ("GM-CSF"), erythropoietin, thrombopoetin granulocyte stimulating factor, ("G-CSF"), macrophage colony stimulating factor ("M-CSF") and stem cell factor ("SCF") to restore platelet and white cell levels, have been used to treat aggressive cancers. Even with the supportive or restrictive therapy, side effects are severe.

Other treatments have been tried in an attempt to improve mortality and morbidity. Vaccines to stimulate the patient's immune system have been attempted, but not with great success. Various cytokines, alone or in combination, such as tumor necrosis factor, interferon gamma, and interleukin-2 ("IL-2") have been used to kill cancers, but have not produced cures. More recently, anti-angiogenic compounds such as thalidomide have been tried in compassionate use cases and shown to cause tumor remission. In animal studies, compounds inducing a procoagulant state, such as an inhibitor of protein C, have been used to cause tumor remission. New studies have shown that soluble cytokine receptors, such as tumor necrosis factor receptors ("TNF-Rs") which are released in a soluble form from tumor cells, in high concentrations relative to normal cells, may restore the immune system's attack on the tumor cells (Jablonska and Peitruska, Arch. Immunol. Ther. Exp. (Warsz) 1997, 45(5-6), 449-453; Chen, et al., J. Neuropathol. Exp. Neurol. 1997, 56(5), 541-550).

U.S. Patent No. 4,708,713 to Lentz describes an alternative method for treating cancer, involving ultrapheresis to remove compounds based on molecular weight, which promotes an immune attack on the tumors by the patient's own white cells.

Selinsky et al (1998) Immunology 94: 88-93 (D1) describes the multifaceted inhibition of anti-tumor immune mechanisms by soluble tumor necrosis factor receptor type 1.

Gatanaga et al (1990) Proc Natl Acad Sci USA 87: 8781-84 (D2) discloses the purification and characterization of an inhibitor (soluble tumor necrosis factor receptor) for tumor necrosis factor and lymphotoxin obtained from serum ultrafiltrates of human cancer patients.

Despite all of these efforts, many patients die from cancer; others are terribly mutilated. It is unlikely that any one therapy will be effective to cure all types of cancer.

It is therefore an object of the present invention to provide a method and system for treatment of solid tumors.

It is a further object of the present invention to provide a method and compositions that does not involve non-selective, extremely toxic, systemic chemotherapy.

### Summary of the Invention

A method to treat disorders characterized by production of soluble TNF receptors, such as many types of cancer, and certain diseases such as HIV, where the disease immunosuppresses the patient, has been developed. As described herein the patient's blood is passed through a column having antibodies immobilized thereon, which bind to and remove the soluble TNF receptor molecules. The process can be performed alone or in combination with other therapies, including radiation, chemotherapy (local or systemic, for example, treatments using alkylating agents, doxyrubicin, carboplatinum, cisplatinum, and taxol, and other drugs which may be synergistic in effect with "unblocked" cytokines: or anti-angiogenic factors. Antibodies are utilized which are immunoreactive with tissue necrosis factor receptor-1 ("TNFR-1"), and tissue necrosis factor receptor-2 ("TNFR-2").

The patient is preferably treated daily for at least three weeks, diagnostic tests conducted to verify that there has been shrinkage of the tumors, then the treatment regime is repeated as needed. The invention is defined by the claims.

### Detailed Description of the Invention

Innate, natural and antigen specific killer mechanisms represent the best arsenal for dealing with melanoma cells *in vitro* and *in vivo*. Central to these cellular destructive mechanisms is tumor necrosis factor (TNF-), an inflammatory cytokine produced by macrophages and earlier mononuclear cells and TNF- , a related cytokine produced and secreted by killerT-lymphocytes with highly selective antigen specific receptors, Old L.J., Antitumor activity of microbial products and tumor necrosis factor, and Bonavida B, et al., (eds): Tumor Necrosis Factor/Cachecin and Related Cytokines, Basell, Karger, 1988. p 7; Haranaka K., et al, Cytotoxic activity of tumor necrosis factor (TNF) on human cancer cells in vitro, Jpn. J Exp. Med. 1981; 51:191; Urban J.L.II, et al., Tumor necrosis factor: A potent effector molecule for tumor cell killing by activated macrophages, Proc. Natl. Acad. Sci. USA 1986; 83-5233; Philip R., et al., Tumor necrosis factor as immunomodulator and mediator of monocyte cytotoxicity induced by itself, Gamma-interferon and Interleukin-1, Nature 1986; 323:86; Ziegler-Heitbrock H.W., et al., Tumor necrosis factor as effector molecule in monocyte-mediated cytotoxicity, Cancer Res. 1986; 46:5947; and Feinman R., et al., Tumor necrosis factor is a important mediator of tumor cell killing by human monocytes, J Immunol. 1987; 138:635. They derive from billions of clones, each with its own specificity. Thus, one clone of these thymus derived lymphocytes gives rise to T-killer (cytotoxic lymphocytes), or other functional classes each with the one specificity of the parent clone. Their mechanisms are related to both antibody dependent and antibody independent cellular tumor toxicity. Receptors for TNF on neoplastic, viral infected, aged cells or those otherwise targeted for destruction can be both a blessing and a curse. In a positive role, they allow binding of TNF to the surface for internalization and destruction of the cell. Unfortunately this receptor hypothesis has a double edge. Certain neoplastic cells such as active melanomas secrete large amounts of these receptors (sTNF-R1 and sTNF-R2) that promptly bind TNF before it can get within the vicinity of the cell, Haranaka K., et al, Cytotoxic activity of tumor necrosis factor (TNF) on human cancer cells in vitro, Jpn. J Exp. Med. 1981; 51:191; Urban J.L.II, et al., Tumor necrosis factor: A potent effector molecule for tumor cell killing by activated macrophages, Proc. Natl. Acad. Sci. USA 1986; 83-5233; Philip R., et al., Tumor necrosis factor as immunomodulator and mediator of monocyte cytotoxicity induced by itself, Gamma-interferon and Interleukin-1, Nature 1986; 323:86; Ziegler-Heitbrock H.W., et al., Tumor necrosis factor as effector molecule in monocyte-mediated cytotoxicity, Cancer Res. 1986; 46:5947; and Feinman R., et al., Tumor necrosis factor is a important mediator of tumor cell killing by human monocytes, J. Immunol. 1987; 138:635. This serves as a defense mechanism on the part of the targeted cell rendering the host immune system ineffective. TNF-R1 and R2 have been characterized with respect to molecular weights (55 and 75 kD respectively), Old L.J., Antitumor activity of microbial products and tumor necrosis factor, and Bonavida B, et al., (eds): Tumor Necrosis Factor/Cachecin and Related Cytokines, Basell, Karger, 1988. p7, Langkopf F., et al., Soluble tumor necrosis factor receptors as prognostic factors in cancer patients, Lancet 1994; 344:57-58; Howard S.T., et al., Vaccinia virus homologues of the Shope fibroma virus inverted terminal repeat proteins and a discontinuous ORF related to the tumor necrosis factor receptor family, Virology 1991; 180:633-664; Mathias S, et al., Activation of the Sphingomyelin signaling pathway intact EL4 cells and in a cell-free system by IL-lb, Science 1993; 259-519-522; and Andrews J.S., et al., Characterization of the receptor for tumor necrosis factor (TNF) and lymphotoxin LT) on human T lymphocytes: TNF and LT differ in their receptor binding properties and the induction of MHC class I proteins on a human CD4+ T cell hybridoma, J. Immunol. 1990;144:2582-2591. They serve to both down regulate the immune response in a normal fashion and overly suppress the immune response as stated above with respect to certain malignancies. They are particularly abundant, and at high level, in patients with melanoma.

### I. Anti-cytokine receptor molecules.

Selective removal or neutralization of the soluble TNF-R1 and TNF-R2 (which function as inhibitors of the cytokine) can be used to promote a selective, safe inflammatory response against a tumor or cells infected with a pathogen such as a virus like HIV or parasite. The neutralizing agent is an antibody reactive with the receptor. The antibodies are reactive with the soluble forms of the receptor. The advantage of selective removal or neutralization is that the same beneficial effect is obtained in treatment of the disorder but the treatment is much less expensive and safer since exogenous plasma or albumin does not have to be administered to the patient when there is selective removal, as in the case of ultrapheresis and the cytotoxic effects of radiation and chemotherapy are avoided.

The receptors are removed by binding to an antibody to the receptor. The antibodies to the receptors can be immobilized in a filter, in a column, or using other standard techniques for binding reactions to remove proteins from the blood or plasma of a patient, or administered directly to the patient in a suitable pharmaceutically acceptable carrier such as saline. As used herein, antibody refers to antibody, or antibody fragments (single chain, recombinant, or humanized), immunoreactive against the receptor molecules. In the most preferred embodiment, the antibody is reactive with the carboxy-terminus of the shed receptor molecules, thereby avoid concerns with signal transduction by the receptor is still present on the cell surface.

Antibodies can be obtained from various commercial sources such as Genzyme Pharmaceuticals. These are preferably humanized for direct administration to a human, but may be of animal origin if immobilized in an extracorporeal device. Antibodies may be monoclonal or polyclonal. The antibodies and device should be sterilized and treated to remove endotoxin and other materials not acceptable for administration to a patient.

Antibodies to the receptor proteins can be generated by standard techniques, using human receptor proteins. Antibodies are typically generated by immunization of an animal using an adjuvant such as Freund's adjuvant in combination with an immunogenic amount of the protein administered over a period of weeks in two to three week intervals, then isolated from the serum, or used to make hybridomas which express the antibodies in culture. Because the methods for immunizing animals yield antibody which is not of human origin, the antibodies could elicit an adverse effect if administered to humans. Methods for "humanizing" antibodies, or generating less immunogenic fragments of non-human antibodies, are well known. A humanized antibody is one in which only the antigen-recognized sites, or complementarily-determining hypervariable regions (CDRs) are of non-human origin, whereas all framework regions (FR) of variable domains are products of human genes. These "humanized" antibodies present a lesser xenographic rejection stimulus when introduced to a human recipient.

To accomplish humanization of a selected mouse monoclonal antibody, the CDR grafting method described by Daugherty, et al., (1991) Nucl. Acids Res. 19:2471-2476 may be used. Briefly, the variable region DNA of a selected animal recombinant anti-idiotypic ScFv is sequenced by the method of Clackson, T., et al., (1991) Nature 352:624-688. Using this sequence, animal CDRs are distinguished from animal framework regions (FR) based on locations of the CDRs in known sequences of animal variable genes. Kabat, H.A., et al., Sequences of Proteins of Immunological Interest, 4th Ed. (U.S. Dept. Health and Human Services, Bethesda, MD, 1987). Once the animal CDRs and FR are identified, the CDRs are grafted onto human heavy chain variable region framework by the use of synthetic oligonucleotides and polymerase chain reaction (PCR) recombination. Codons for the animal heavy chain CDRs, as well as the available human heavy chain variable region framework, are built in four (each 100 bases long) oligonucleotides. Using PCR, a grated DNA sequence of 400 bases is formed that encodes for the recombinant animal CDR/human heavy chain FR protection.

The immunogenic stimulus presented by the monoclonal antibodies so produced may be further decreased by the use of Pharmacia's (Pharmacia LKB Biotechnology, Sweden) "Recombinant Phage Antibody System" (RPAS), which generated a single-chain Fv fragment (ScFv) which incorporates the complete antigen-binding domain of the antibody. In the RPAS, antibody variable heavy and light chain genes are separately amplified from the hybridoma mRNA and cloned into an expression vector. The heavy and light chain domains are co-expressed on the same polypeptide chain after joining with a short linker DNA which codes for a flexible peptide. This assembly generated a single-chain Fv fragment (ScFv) which incorporates the complete antigen-binding domain of the antibody. Compared to the intact monoclonal antibody, the recombinant ScFv includes a considerably lower number of epitopes, and thereby presents a much weaker immunogenic stimulus when injected into humans.

The antibodies can be formulated in standard pharmaceutical carriers for administration to patients in need thereof. These include saline, phosphate buffered saline, and other aqueous carriers, and liposomes, polymeric microspheres and other controlled release deliver devices, as are well known in the art. The antibodies can also be administered with adjuvant, such as muramyl dipeptide or other materials approved for use in humans (Freund's adjuvant can be used for administration of antibody to animals).

In the preferred embodiment, antibodies are immobilized to a solid support, such as the SEPHAROSE™ column in the examples, using standard techniques such as cyanogen bromide or commercially available kits for coupling of proteins to membranes formed of materials such as nitrocellulose or polycarbonate.

Treatment is conducted over a period of time until a positive indication is observed. This is typically based on diagnostic tests which show that there has been some reduction in tumor size or which suggests tumor inflammation. The patient is preferably treated daily for three weeks, diagnostic tests conducted to verity that there has been shrinkage of the tumors and/or inflammation, then the treatment regime is repeated.

Surgical (or vacuum) removal of necrotic material may be required prior to or during treatment to avoid toxicity associated with high tumor burden.

### II. Treatment with Adjuvant Therapies

It would clearly be advantageous to cause complete remissions. Based on the presumed mechanism that the process is removing immune inhibitors produced by the tumors, especially inhibitors of cytokines and other immune mediators, it is possible to treat the patients with adjuvant or combination therapies, that enhance the results achieved with the antibodies to TNF receptors. These include anti-angiogenic compounds, such as thalidomide, procoagulant compounds, cytokines and other immunostimulants. Standard chemotherapeutic agents and/or radiation can also be used with the ultrapheresis with the antibody treatment.

### A. Anti-Angiogenic Compounds

Any anti-angiogenic compound can be used. Exemplary anti-angiogenic compounds include O-substituted fumagillol and derivatives thereof, such as TNP-470, described in U.S. Patent Nos. 5,135,919, 5,698,586, and 5,290,807 to Kishimoto, et al.; angiostatin and endostatin, described in U.S. Patent Nos. 5,290,807, 5,639,725 and 5,733,876 to O'Reilly; thalidomide, as described in U.S. Patent Nos. 5,629,327 and 5,712,291 to D'Amato; and other compounds, such as the anti-invasive factor, retinoic acid, and paclitaxel, described in U.S. Patent No. 5,716,981 to Hunter, et al., and the metalloproteinase inhibitors described in U.S. Patent No. 5,713,491 to Murphy, et al. Thalidomide is administered once daily, 200 mg orally.

### B. Procoagulant Compounds

Protein C is a vitamin K-dependent plasma protein zymogen to a serine protease. Upon activation it becomes a potent anticoagulant. Activated protein C acts through the specific proteolysis of the procoagulant cofactors, factor VIIIa and factor Va. This activity requires the presence of another vitamin K-dependent protein, protein S, calcium and a phospholipid (presumably cellular) surface. As described in Hemostasis and Thrombosis: Basic Principles and Clinical Practice 2nd Ed., Colman, R.W., et al.,p. 263 (J.B.Lippincott, Philadelphia, PA 1987), protein C circulates in a two-chain form, with the larger, heavy chain bound to the smaller light chain through a single disulfide link. Protein C is activated to activated protein C (APC). Thrombin is capable of activating protein C by the specific cleavage of the Arg¹²-Leu¹³ bond in the heavy chain. *In vivo*, in the presence of physiological concentrations of calcium, the rate of this activation is enhanced dramatically when thrombin is bound to the endothelial cell cofactor, thrombomodulin. Matschiner, et al., Current Advances in Vitamin K Research, pp. 135-140, John W. Suttie, ed. (Elsevier Science Publishing Co., Inc. 1988) have further reviewed the role of the Vitamin K dependent proteins in coagulation.

Blockage of the natural anticoagulant pathways, in particular the protein C pathway, uses the natural procoagulant properties of the tumor to target the tumor capillaries for microvascular thrombosis, leading to hemorrhagic necrosis of the tumor, as described in U.S. Patent No. 5,147,638 to Esmon, et al. Examples of such compounds include anti-protein C and anti-protein S.

### C. Cytokines

The biologic activity and clinical effectiveness of pro-inflammatory cytokines is augmented by ultrapheresis in the patient with cancer and other states of acquired immune tolerance. Specifically, both TNF alpha and TNF beta, in doses of between approximately 100 to 500 micrograms per meter squared body surface area (M2BSA), can enhance the immune reaction in aggressive tumors. Monocyte and lymphocyte activation is augmented by INH-alpha, INF-beta and gamma. The IL-1 and IL-2 receptor antagonists are removed by ultrapheresis and thereby upregulate the *in vivo* activity of these cytokines. An 80 kD glycoprotein, which is responsible for inhibiting blastoid transformation in advanced malignancy, chronic infectious disease and pregnancy, has recently been found, and appears to be responsible for the loss of delayed hypersensitivity reactions in these diseases, which is removed by this process. This is significant because in removing this type of suppression, vaccines of all types will work better. Dosage regimes for IFN-alpha and beta arc 3 M units subcutaneous three times a week up to 20 M units/M2 BSA daily. Interferon-gamma is administered in a dosage of between 100 to 1000 micgms per day.

### D. Chemotherapeutic Agents

Preferred chemotherapeutic agents are those which are synergistic with TNF, for example, alkylating agents, doxyrubicin, carboplatinum, cisplatinum, and tomoxifen. Tamoxifen plays a role not only in blocking of estrogen receptors but also certain growth factor receptors such as epidermal derived growth factor ("EDGF"), fibroblast derived growth factor ("FDGF"), tumor derived growth factor ("TDGF"), TDGF-β and platelet derived growth factor ("PDGF") and therefore may be complementary to inflammation against cancers provoked by ultrapheresis.

### E. Radiation

Radiation therapy is destructive of normal tissue, causing tumors to die partially by an inflammatory attack. Ultrapheresis allows the use of lower doses of radiation to kill residual tumor cells and spare normal tissue. In a preferred method, ultrapheresis is used as the initial therapy, followed by radiation at approximately one-half of the normal dosages. It is well established that TNF kills tumor cells by generating free oxygen radicals, hydroxyl radicals and halide ions, and that radiation therapy generates carbonium ions in tissue. Therefore the combination of the two is more effective in killing cancer cells than either alone.

### III. Examples

### Example 1: Treatment of a Patient with Ultrapheresis having antibodies immobilized on the Filter.

### Materials and Methods

Monoclonal antibody was obtained from R&D Systems, Minneapolis, MN, and purified for administration to a patient. This antibody is reactive with TNF R1 and R2 inhibitors.

A filtration system was assembled using an Eva Flux 4 A filter as the primary filter to remove ultrafiltrate containing these inhibitors from the cancer patient's blood. Monoclonal antibody in a dose of 1 mg per liter of normal ultrafiltrate of the monoclonal antibody and 1 mg of the $2 monoclonal antibody were added to that replacement solution. In this circuit the ultrafiltrate of the initial 4 A filter was delivered by a separate blood pump to a Kuraray 3 A filter. The retentate of the 3A filter was then discarded and the ultrafiltrate of the 3A filter was metered back into the filtered blood from the 4 A filter as replacement solution. To make the discard; i.e., the retentate of the 3 A filter, normal ultrafiltrate with monoclonal antibody added to it was metered into the intra circuit between the 4 A and 3 A filters.

### Results

Addition of the monoclonal antibodies to ultrafiltrated cancer sera that possess elevated levels of the inhibitors decreases the level of detectable inhibitor by Elias Assay to zero.

Addition of the monoclonal antibodies to the replacement fluid following ultrapheresis led to an increased reduction of both the soluble receptor to TNF R1 and R2 in the ultrafiltrate of the second filter.

The purpose of this was to test whether or not this murine monoclonal antibody could capture the inhibitor and aid in its removal from blood since the complex of antibody and antigen could not pass through the pores of the 3 A filter and thus be discarded in the retentate of the 3A filter. This was considerably more effective than the single separation technique and replacement with normal ultrafiltrate. There was also a heightened tumor specific inflammatory response by doing this and an increased rate of tumor destruction. These experiments strongly indicate that the monoclonal antibody, preferably humanized to 97% to 99% human form by substituting human constant regions for human constant regions on the antibody, preserve its capturing and neutralizing capability with the murine variable regions of the antibody and use the antibody as the therapeutic drug in clinical trials with a very high expectation that it would neutralize soluble receptors to TNF and cause tumor destruction in a human.

### Example 2: Treatment of a Patient with mAb to TNF Receptors.

A patient with vaginal metastasis of colon cancer was treated for one week with a three hour infusion of monoclonal antibody to TNF receptor 1 and TNF receptor 2. This led to a 75% reduction in the tumor size within one week.

### Example 3: Treatment of Melanoma Patient

A procedure is described in case report form, that utilizes apheresis and immunological affinity chromatography to treat a melanoma patient with short term need and weakening long term prognosis.

Previous studies utilizing ultrafiltration, with selective pore sieving by passing patient's plasma through cartridges, have been shown to reduce sTNF-R1 and R2 levels. The period of this procedure seems to be of sufficient length to allow TNF to rebound and selectively produce apoptosis or membrane disarray of melanoma cells, Gatanaga T., et al., Identification of TNF-LT blocking factor(s) in the serum and ultrafiltrates of human cancer patients, Lymphokine Res. 1990;9:225-9. Instead of using ultrafiltrate cartridges, this apheresis system was coupled to Sepharose® gel columns in parallel, one of which contained monoclonal human anti TNF-R1 and the second anti TNF-R2. The concept of affinity chromatography preparations has been technically available for protein separation and purification, and improved upon over the past 30 years, Ey, P.L., et al., Isolation of pure IgG1, IgG2a, and IgG2b. immunoglobulins from mouse serum using protein A-Sepharose, Immunochemistry 1978;15:429-436. This type of device represents one of the few examples of linking *in vivo* production of TNF inhibitors to *in vitro* removal and return of the purified extracted plasma to the patient to prevent fluid reduction.

The patient is a 55 year old Russian gentleman with metastatic melanoma. The patient smoked 2-3 packs of cigarettes a day for some 20 years. He quit this habit several years ago. He was also a heavy alcohol user in years past but had decreased his intake to 1-2 glasses of wine a day. Review of his medications on this date revealed methylprednisolone 4 mg in AM and 4 mg in PM. Apparently this was being taken as replacement therapy for adrenal cortical suppression that was graded iatrogenically at the time of the treatment of his alveolitis (see below). He was additionally taking narcotic analgesics. As a child he suffered the usual childhood diseases, denies rheumatic fever, scarlet fever or diphtheria. As an adult he has had no major medical illnesses save those described above. He has had no other major surgeries in the past and has no known allergies.

His history of present illness began in November of 1995 when he noted growth of a right facial naevus which bled and enlarged over the period of one year. This was treated initially by cryotherapy. It regrew within two months and was excised. Histology was that of a malignant melanoma (Clark's level unknown). Staging work up at the time was negative and included CT scans of the head, neck, chest and abdomen. He remained disease free until March of 1996 when he developed right cervical and right submental adenopathy. Preoperative CT scan of the head, neck, chest and abdomen confirmed the right cervical adenopathy but revealed no other sites of metastases. In June of 1996 he underwent re-excision with a right radical neck dissection. In this material, one lymph node was histologically confirmed to involve melanoma. The patient was treated with a course of Vindesine 3 mg/m² every three weeks, Dacarbazine 100 mg/m² every three weeks for four cycles. He subsequently developed cutaneous metastases in the skin of his left shoulder, multiple metastases to the scars within the left anterolateral neck and multiple axillary metastases treated with fifteen subsequent excisions of recurrent metastases. In March of 1999 he was offered a trial of Interleukin-2 but on this developed severe pulmonary toxicity that had a protracted course and was diagnosed as idiopathic fibrosing alveolitis. Interleukin-2 was discontinued and he received radiation therapy to his right neck and axilla for six weeks beginning in the month of May 1999. He developed low back pain in August of 1999. Work up in October of 1999 revealed bone metastasis of the vertebral body of T-11 and subsequent MRI revealed a lytic destructive process in the right transverse process and pedicle of the 11^{th} thoracic vertebra, as well as complete replacement of the vertebral body at T-11. Additional metastases were appreciated in the vertebral body of the 9^{th} thoracic vertebral as well as the 10^{th}. Also there was involvement of L-1 and L-2 vertebral bodies. Tumor seen again on the March 16, 2000 MRI revealed growth posteriorly from the mid body of the 11^{th} thoracic vertebral into the spinal canal by 7.4 to 7.8 mm with posterior displacement of the spinal cord. CT scan of the chest, abdomen and pelvis revealed possible multiple liver metastases but no other suggestion of visceral metastases.

The patient was then considered for a trial of UltraPheresis™ in an effort to reduce solubilized receptors to tumor necrosis factor, both sTNF-R1 and sTNF-R2. As facilities for the application of this form of semi-selective plasma exchange did not exist in Moscow at this time, affinity column separation of inhibitors was explored. Monoclonal antibodies against sTNF-R1 and R2 delivered to the Cardiology Research Center in Moscow for Dr. Sergei N. Petrovsky, PhD, head of the group for Affinity Sorbents for Medicine, Pocard, Ltd, 3-rd Cherepkovskaya str., 15a, Moscow, 121552, Russia. Ninety milligrams of anti sTNF-R1 monoclonal antibody and 180 mg of anti sTNF-R2 monoclonal antibody were then bound with sterile Sepharose® using cyanogen bromide in a glass column previously described for use in the lipopack cholesterol absorbent column technology. The particular methodology used is well described and is commercially available in Russia for the development of these LDL absorbent columns. The columns were prepared under sterile conditions in a GSIO 9,001 facility. They were subjected to endotoxin testing, viral, fungal and bacterial cultures, and prepared for human use under written Informed Consent and under approval of the Kremlin President's Hospital Medical Center.

On May 2nd, 2000 the patient's physical examination was that of a well-developed, well-nourished male who appeared his stated years. Examination of his head revealed a normal hair distribution and texture. His tympanic membranes and external auditory canals were clear. The sclerae and conjunctivae were clear. The pupils were round, reactive to light and accommodation. EOM intact. Funduscopic examination was normal. He had a healed graft over his right inferior cheek and extensive scarring over the right anterolateral neck consistent with his history of prior right radical neck dissection. There were no demonstrable pathologic masses within the skin, the scar, or pathologic nodes appreciated either in the cervical nodes or the supraclavicular fossae bilaterally. His lungs were clear to ausculation and percussion. His precordium demonstrated a non-displaced PMI, a normal S 1 and S2 without gallop, murmur or rub. With the right arm exhibited there was 3+lymphedema. The right axilla was poorly examined due to extensive scarring in that area but no palpable nodes were appreciated. His abdomen was mildly obese. His liver and spleen were normal to physical examination. His axillary lymphatics were unremarkable. The gcnitalia was that of a normal mature male without pathologic mass. The lower extremities revealed no edema, cyanosis or clubbing and exhibited full ROM. His neurologic examination included a normal mental status. Cranial nerves 2-12 were intact. His DTR's were 2+ and symmetric. Motor and sensory testing was normal. His cerebellar examination revealed no dysmetria, dysarthria or dysdiadochokinesia. He was essentially confined to bed due to back pain only, but was able to roll from left to right without assistance. He had been confined to a wheelchair for the antecedent two months due to back pain and was wearing a back brace which was removed for physical exam.

His laboratory parameters included a hemoglobin of 8.8 gms, WBC 2,800 with normal differential. His platelet count was 121,000. The comprehensive metabolic panel was unremarkable and alkaline phosphatase was normal.

An MRI scan of the patient's 11th thoracic vertebral body revealed a mass placing pressure on the spinal cord. This was taken during the week prior to intensive therapy started in April of 2000 and continuing through May.

On the first day an 18 gauge plastic cannula was inserted in the left antecubital vein. A second was established in the right greater saphenous vein of the leg. The patient was connected to a standard Cobe Spectra centrifically based plasma separator. Six hundred cc's of plasma was then harvested and replaced with 5% albumin in saline. The patient's plasma was then pumped over column one which contained 45 mg of anti sTNF-R1 monoclonal antibody and then passed to column two which contained 90 mg of anti sTNF-R2 monoclonal antibody. The material eluted from the column was then analyzed for the level of each inhibitor still in the plasma and 50 cc's of that plasma was then injected into the patient at the end of pheresis to look for any febrile reactions or allergic reactions. He tolerated this with no apparent clinical adverse effect.

Subsequent analyses of the patient's plasma and the eluate of the column revealed that the column was able to capture essentially all of the inhibitor presented to it in this 600 ml plasma volume. The patient was maintained in the hospital over night and on the morning of the 4th of May, he was brought from hospital room back to the apheresis suite. He had a comfortable evening and ate a normal dinner and breakfast. The IV's were re-established in the same sites. The patient was re-attached to the Cobe Spectra machine and on this date, 3 liters of plasma was harvested and delivered to the columns as described above in a continuous fashion until 3 liters of plasma was treated.

His R1 level before treatment was 1500 and after treatment was 1450. His R2 level before treatment was 5000 and after treatment was 3800 on this date. Again he tolerated the procedure well with no clinical adverse effect and no increase in pain in his back.

On the third day the 6th of May, the treatment was repeated. Three liters of plasma were again pheresed over the columns in an identical fashion as described above. His pretreatment R1 was 2300, post treatment R1 was 1600. Pretreatment R2 was 5200, post treatment R2 was 3200. At the end of each treatment the columns were washed with glycine buffer at a pH of 2.5 to elute the bound inhibitor from them and measure them quantitatively. It was determined that at these amounts of treated plasma the columns were not saturated and significant quantities of inhibitor removed.

His fourth treatment was on the 7th of May. He was increased to 4 liters of treated plasma. The procedures were repeated each day with gradual escalations in amount of plasma treated to a maximum treated plasma of 8 liters on the May 10th, 11th, 12th, 13th, and 14th. On May 16^{th}, two columns were used in parallel, thus increasing the amount of plasma delivered to each column remained at 30 mls per minute, for a total of 60 mls of plasma per minute. This resulted in a pretreatment R1 of 2600 and a post treatment of 1700. R2 pretreatment was 4250 and went to post treatment of 2700.

He was subsequently treated with 8 liters of plasma a day using the double column method. On the 21 st of May he had a repeat CAT scan of his spine which revealed complete resolution of tumor. Three days after that, May 24th,he had a repeat MRI which was compared to the pretreatment MRI and confirmed a complete response. The patient was followed carefully in the hospital by his attending physicians as well as attending neurosurgeons, who followed him on a daily basis concerned about tumor bleeding or tumor swelling in his tight and anatomically dangerous places but fortunately the patient enjoyed a complete response with no apparent adverse effect.

For the details of daily treatment in terms of volumes, columns, blood flow rates and plasma flow rates see Table 1.

The patient has enjoyed an apparent complete response without any significant adverse effect. He was able to get up and walk after the fourth procedure. Two additional courses were planned in an endeavor to consolidate this response. This case is consistent with the observations that a salutatory tumor response can be achieved in melanoma by removing solubilized receptors to TNF. This column is so specific that it removes only sTNF- R1 and R2 and that is the only explanation for the response that this man has had from an oncologic point of view. A profound column yield was observed on the third treatment day for sTNF-R2 with modulation for the remaining treatment days throughout this fifteen day course. R1 peaked on treatment day 7 with the total amount removed of 6 million pg. This also modulated throughout the course of treatments but never approached the 16 million mark set by sTNF-R2.

Radiographic examination on the day following his first fifteen day course of apheresis with anti R1 and anti R2 affinity column extraction revealed no melanoma and considerable reduction of the lesion at the 4th lumbar vertebral body. Currently the patient remains active, with good appetite, is walking normally and his back pain is much improved. He has positive anticipation for his second course of apheretic treatments.

### Example 4: Production of Polyclonal Antibodies to STNF R1 and R2; Preparation of Column for Treatment of Patients

Polyclonal antibodies were produced in New Zealand white rabbits injected with recombinant antigen, soluble tumor necrosis factor receptor ("STNF") R1 and R, injected into the rabbit on a standard immunization protocol, then boosted. 200 mg of polyclonal antibody may be produced against STNF R1 and R2, per liter. The animals will be bled monthly. 200 mg of antibody can be bound safely to 200 mg of AH SEPHAROSE^{™} beads. The binding is done with ethanolamine and periodate. Binding is therefore excellent. This matrix is then placed in a 200 mg polycarbonate column. Each step is done in an aseptic fashion and the final product is then terminally sterilized with standard radiation protocols and subjected to USDA standard testing for pyrogen and infectious agents.

This amount of antibody is enough to remove STNF R1 and STNF R2 in human extracellular water sufficient to reduce the level of 10,000 pg per ml to under 1,000 pg per ml in two to three hours of plasma exchange.

The use of the columns to reduce inhibitor levels to less than 1000 pg/ml over a period of at least three weeks has resulted in remissions of between 40 and 90% in non-small cell lung cancer, breast cancer and melanoma patients. It is therefore predictable that the treatment results in a rather consistent tumor specific inflammatory response and the majority of patients having the most common tumor types, including breast, small cell lung, colon, ovarian, hepatic, melanoma, and renal cell carcinoma as well as ovarian and endometrial cancers should respond to the treatment. In combination with antibodies against vascular endothelial growth factor receptor and/or epidermal growth factor receptor and/or antibodies against fibroblast derived growth factor and transforming growth factor receptor, either singularly or in combination, the treatment is expected to produce excellent responses in these tumor types and may play a role in the clinical management of hematopoietic disorders as well.

The methods and systems disclosed herein are useful for treatment of patients with cancer, immune-mediated disorders, chronic parasitism, some viral diseases especially viral diseases such as HIV which cause immunosuppresion, and other disorders characterized by elevated levels of TNF receptors or inhibitors to IL-2, IL-6, gamma interferon, or other pro-inflammatory signals as well as white cell activation. An example demonstrates efficacy in treating a cancer patient.

## Claims

1. An extracorporeal device through which a patient's blood or plasma can be circulated prior to being returned to the patient,
said device having immobilised therein antibodies that selectively bind to soluble TNF receptor 1 molecules and antibodies which bind to soluble TNF receptor 2 molecules,
and wherein binding of the antibodies to the soluble TNF receptor molecule removes the soluble TNF receptor molecule from the patient's blood or plasma.

2. The extracorporeal device of Claim 1, wherein the antibodies and the device have been sterilized and endotoxin has been removed from the antibodies and the device.

3. The extracorporeal device of any of Claims 1 or 2, wherein the device is an adsorbent column that selectively removes said soluble cytokine receptor molecules from the blood or plasma.

4. The extracorporeal device of Claim 3, wherein the column can adsorb the soluble TNF receptors present in 600 ml of plasma.

5. The extracorporeal device of Claim 3, wherein the column can adsorb the soluble TNF receptors present in 4,000 ml of plasma.

6. The extracorporeal device of any of Claims 1 or 2, wherein the device comprises a filter on which the antibodies are immobilised.

7. The extracorporeal device according to any of Claims 1-6 further comprising a primary filter or centrifuge to separate ultrafiltrate or plasma from the blood of a patient.

8. The extracorporeal device according to any of Claims 1-7 further comprising a cannula and tubing suitable for connecting the device to the patient.

9. The extracorporeal device according to any of Claims 1-8, wherein the antibodies are monoclonal antibodies, antibody fragments or single chain antibodies.

10. Use of antibodies that selectively bind to soluble TNF receptor 1 molecules and of antibodies which bind to soluble TNF receptor 2 molecules, in the manufacture of an extracorporeal device having said antibodies immobilised therein for inducing an immune response against transformed, infected or diseased tissue in a patient by circulating the patient's blood or plasma through the device such that said antibodies bind to the soluble cytokine receptor molecules in the patient's blood or plasma, thereby removing the soluble TNF receptor molecules from the patient's blood or plasma, and returning the blood or plasma to the patient.

11. The use of Claim 10, wherein the antibodies and the device have been sterilized and endotoxin has been removed from the antibodies and the device.

12. The use of any of Claims 10 or 11, wherein the device is an adsorbent column that selectively removes said soluble cytokine receptor molecules from the blood or plasma.

13. The use of Claim 12, wherein the column can adsorb the soluble TNF receptor present in 600 ml of plasma.

14. The use of Claim 12, wherein the column can adsorb the soluble TNF receptor present in 4,000 ml of plasma.

15. The use of any of Claims 10 or 11, wherein the device comprises a filter on which the antibodies are immobilised.

16. The use of any of Claims 10 to 15, wherein the antibodies are monoclonal antibodies, antibody fragments or single chain antibodies.

## Patentansprüche

1. Extrakorporale Vorrichtung, durch die Blut oder Plasma eines Patienten zirkuliert werden kann, bevor es zu dem Patienten zurückgeführt wird, wobei die Vorrichtung darin immobilisierte Antikörper hat, die lösliche TNF-Rezeptor-1-Moleküle selektiv binden, und Antikörper, die lösliche TNF-Rezeptor-2-Moleküle binden, und wobei das Binden der Antikörper an die löslichen TNF-Rezeptor-Moleküle die löslichen TNF-Rezeptor-Moleküle aus dem Blut oder Plasma des Patienten entfernt.

2. Extrakorporale Vorrichtung nach Anspruch 1, wobei die Antikörper und die Vorrichtung sterilisiert wurden, und Endotoxin von den Antikörpern und der Vorrichtung entfernt wurde.

3. Extrakorporale Vorrichtung nach einem beliebigen der Ansprüche 1 oder 2, wobei die Vorrichtung eine adsorbierende Säule ist, die die löslichen Zytokin-Rezeptormoleküle selektiv aus dem Blut oder Plasma entfernt.

4. Extrakorporale Vorrichtung nach Anspruch 3, wobei die Säule die löslichen TNF-Rezeptoren adsorbieren kann, die in 600 ml Plasma vorliegen.

5. Extrakorporale Vorrichtung nach Anspruch 3, wobei die Säule die löslichen TNF-Rezeptoren adsorbieren kann, die in 4.000 ml Plasma vorliegen.

6. Extrakorporale Vorrichtung nach einem beliebigen der Ansprüche 1 oder 2, wobei die Vorrichtung einen Filter umfasst, auf dem die Antikörper immobilisiert sind.

7. Extrakorporale Vorrichtung nach einem beliebigen der Ansprüche 1 bis 6, weiterhin umfassend einen Primärfilter oder eine Zentrifuge, um Ultrafiltrat oder Plasma aus dem Blut eines Patienten abzutrennen.

8. Extrakorporale Vorrichtung nach einem beliebigen der Ansprüche 1 bis 7, weiterhin umfassend eine Kanüle und Schlauchmaterial, geeignet zum Verbinden der Vorrichtung mit dem Patienten.

9. Extrakorporale Vorrichtung nach einem beliebigen der Ansprüche 1 bis 8, wobei die Antikörper monoklonale Antikörper, Antikörperfragmente oder Einzelkettenantikörper sind.

10. Verwendung von Antikörpern, die lösliche TNF-Rezeptor-1-Moleküle selektiv binden, und von Antikörpern, die lösliche TNF-Rezeptor-2-Moleküle binden, in der Herstellung einer extrakorporalen Vorrichtung mit darin immobilisierten Antikörpern zum Induzieren einer Immunantwort gegen transformiertes, infiziertes oder erkranktes Gewebe in einem Patienten durch Zirkulieren des Patientenbluts oder Plasmas durch die Vorrichtung, so dass die Antikörper die löslichen Zytokin-Rezeptormoleküle in dem Patientenblut oder Plasma binden, wodurch die löslichen TNF-Rezeptor-Moleküle aus dem Patientenblut oder Plasma entfernt werden, und Zurückführen des Blutes oder des Plasmas zu dem Patienten.

11. Verwendung nach Anspruch 10, wobei die Antikörper und die Vorrichtung sterilisiert wurden, und Endotoxin von den Antikörpern und der Vorrichtung entfernt wurde.

12. Verwendung nach einem beliebigen der Ansprüche 10 oder 11, wobei die Vorrichtung eine absorbierende Säule ist, die lösliche Zytokin-Rezeptormoleküle aus dem Blut oder Plasma selektiv entfernt.

13. Verwendung nach Anspruch 12, wobei die Säule den löslichen TNF-Rezeptor adsorbieren kann, der in 600 ml Plasma vorliegt.

14. Verwendung nach Anspruch 12, wobei die Säule den löslichen TNF-Rezeptor adsorbieren kann, der in 4.000 ml Plasma vorliegt.

15. Verwendung nach einem beliebigen der Ansprüche 10 oder 11, wobei die Vorrichtung einen Filter umfasst, auf dem die Antikörper immobilisiert sind.

16. Verwendung nach einem beliebigen der Ansprüche 10 bis 15, wobei die Antikörper monoklonale Antikörper, Antikörperfragmente oder Einzelkettenantikörper sind.

## Revendications

1. Un appareil extracorporel par lequel du sang ou du plasma d'un patient peut circuler avant d'être retourné vers le patient, appareil dans lequel sont immobilisés des anticorps qui se lient sélectivement à des molecules d'un récepteur 1 de TNF soluble et des anticorps qui se lient à des molécules d'un récepteur 2 de TNP soluble, et dans lequel la liaison des anticorps aux molécules du récepteur de TNF soluble retire la molécule du récepteur de TNF soluble du sang ou plasma du patient.

2. L'appareil extracorporel selon la revendication 1, dans lequel les anticorps et l'appareil ont été stérilisés et l'endotoxine a été enlevée des anticorps et de l'appareil.

3. L'appareil extracorporel selon une quelconque des revendications 1 ou 2, dans lequel le dispositif consiste en une colonne d'adsorbant qui retire de façon sélective du sang et plasma lesdites molécules de récepteur de cytokine.

4. L'appareil extracorporel selon la revendication 3, dans lequel la colonne peut adsorber les récepteurs de TNF solubles présents dans 600 ml de plasma.

5. L'appareil extracorporel selon la revendication 3, dans lequel la colonne peut adsorber les récepteurs de TNF solubles présents dans 4 000 ml of plasma.

6. L'appareil extracorporel selon l'une des revendications 1 et 2, dans lequel l'appareil comprend un filtre sur lequel les anticorps sont immobilisés.

7. L'appareil extracorporel selon une quelconque des revendications 1 à 6, comprenant en outre un filtre primaire ou centrifuge pour séparer l'ultrafiltrat ou plasma du sang d'un patient.

8. L'appareil extracorporel selon une quelconque des revendications 1 à 7, comprenant en outre un cathéter et une tubulure convenables pour connecter l'appareil au patient.

9. L'appareil extracorporel selon une quelconque des revendications 1 à 8, dans lequel les anticorps sont des anticorps monoclonaux, des fragments d'anticorps, ou des anticorps à chaîne unique.

10. L'utilisation d'anticorps qui se lient sélectivement à des molecules d'un récepteur 1 de TNF soluble et d'anticorps qui se lient à des molécules d'un récepteur 2 de TNP soluble, dans la fabrication d'un appareil extracorporel dans lequel sont immobilisés des anticorps pour induire une réponse immunitaire à l'égard de tissus transformés, infectés ou malades d'un patient qui consiste à faire circuler le sang ou plasma du patient dans ledit appareil de façon que lesdits anticorps se lient aux molécules du récepteur de cytokine soluble du sang ou plasma du patient, et ainsi de retirer du sang ou plasma du patient les molécules du récepteur de TNF soluble puis à retourner le sang ou plasma au patient.

11. L'utilisation selon la revendication 10, dans laquelle les anticorps et l'appareil ont été stérilisés et l'endotoxine a été enlevée des anticorps et de l'appareil.

12. L'utilisation selon une quelconque des revendications 10 ou 11, dans laquelle l'appareil consiste en une colonne d'adsorbant qui retire de façon sélective lesdites molécules de récepteur de cytokine du sang et du plasma.

13. L'utilisation selon la revendication 12, dans laquelle la colonne peut adsorber les récepteurs de TNF solubles présents dans 600 ml de plasma.

14. L'utilisation selon la revendication. 12, dans laquelle la colonne peut adsorber les récepteurs de TNF solubles présents dans 4,000 ml de plasma

15. L'utilisation selon une quelconque des revendications 10 ou 11, dans laquelle l'appareil comprend un filtre sur lequel les anticorps sont immobilisés.

16. L'utilisation selon une quelconque des revendications 10 à 15, dans laquelle les anticorps sont des anticorps monoclonaux, des fragments d'anticorps, ou des anticorps à chaîne unique.
